# EUROPEAN PATENT APPLICATION

(11) **EP 4 527 316 A1**
(43) Date of publication of application: **26.03.2025**
(21) Application number: 22965686.3
(22) Date of filing: 15.11.2022
(51) Int. Cl.: A61B 17/00, A61M 25/00

(54) **DEVICE FOR TREATING THE SAPHENOUS VEIN AND RELATED VARICOSE VEINS AND A METHOD FOR USING SAME IN THE HUMAN BODY**

(71) Applicant: FUNDACION SANTA FE DE BOGOTA, Bogota, 11001000 (CO)
(72) Inventor: ULLOA, Jorge, Bogota, 11001000 (CO)
(74) Representative: Cueto, Sénida
(86) International application number: PCT/IB2022/060949
(87) International publication number: WO 2024/105430

(57) **Abstract**

The invention relates to an intravenous type device for the treatment of varicose veins consisting of a body (1), a working end (2) towards the inner part of the patient's leg with an exit hole (3), a working end (4) manipulated by medical personnel consisting of three routes of admission, the first of them (5) to enter a fiber optic element, two other ports (6, 7) that can enter an inflatable balloon device (8) and a sclerosing foaming agent such as polidocanol (9), the main body (1) also presents a plurality of windows or orifices (10).

## Description

### FIELD OF INVENTION

The invention relates to a three-way device for the intravenous treatment of the saphenous vein and associated varicose veins.

### BACKGROUND TO THE INVENTION

In recent years, surgical procedures, sclerotherapy and especially intravenous procedures have become increasingly important in the treatment of varicose veins. These procedures are effective for the ablation of the associated saphenous veins and varicose veins and have fewer side effects for patients than conventional surgical procedures.

Ablation of superficial veins that have lost their ability to pump blood to the heart has known beneficial therapeutic effects. For example, the use of adhesives to stop the flow into veins forming a permanent hardened occluded mass is limited due to the risk of letting the adhesive enter the deep system. The migration of the adhesive to undesirable, otherwise healthy sections can have very serious effects. For example, using cyanoacrylate to occlude a large saphenous vein, a usual vein closure procedure could result in the migration of the adhesive to the deep system, for example, into the common femoral vein. If cyanoacrylate migrates into the deep system, it will harden and obstruct the deep vein; a clot will form proximal and distal to the glue mass and an uncorrected immobile deep vein thrombosis (DVT) will result. Pain and swelling will occur in the affected limb, and the veins will be dilated (The clot may break off and travel to the lungs, causing a pulmonary embolism (PE) associated with a substantial mortality rate.) Emergency medical treatment of acute deep vein thrombosis is usually treated by breaking down the clot using thrombolytic agents and anticoagulation (blood thinning). However, in this case, the hardened glue mass cannot be removed. There is no obvious way to correct the condition. An attempt can be made to surgically bypass the hardened mass, but these procedures are known to be dangerous and have a limited success rate. For example, application WO2013090563A1 uses an intraluminal element, such as a rotating wire catheter (dispersion tip), is where an adhesive is delivered to the veins and arteries while controlling the migration of the adhesive. The tip of the rotating dispersion together with a sclerosing substance and adhesive or adhesive alone produces the vasspasm. Consistently and reliably, the vein shows perimeter narrowing and a noticeable reduction in diameter resulting in complete occlusion of the treated vein at the treatment site. With spasm, the vein is occluded distally and at the adhesive injection site, thereby preventing adhesive migration and collateral damage. The spasm blocks the flow of adhesive into the deep system and also reduces the volume of adhesive needed. It provides a more effective and reliable vein occlusion. There is no need to block the flow into the deep system using external compression or any other method, such as a mechanical plug or balloon. Obstruction of the flow by a spasm caused by the device makes a safer system possible.

It is also known in the state of art document US10300250B2**,** which provides a device with a simpler design that allows the effective treatment of varicose veins. The device that solves this problem comprises a catheter, which has a balloon with at least one cutting element ("cutting balloon") and which has side openings. A sclerosing agent can enter the vein through these lateral openings. Using the "cutting balloon," at least part of the inner wall of the vein can be cut to cause destruction of the intima and middle of the vein. The device thus allows treatment in the form of ablation with an intravenous mechanical chemo catheter. As a result of the combination of sclerotherapy with mechanical destruction of insufficient veins, the efficacy of treatment may be increased compared to sclerotherapy alone. Compared to conventional intravenous procedures, the additional use of expensive supplementary equipment is not required. Overall, the design of the device according to the invention is relatively simple and the equipment requirements can be reduced, making possible a treatment that is efficient, as well as economical in time and money.

However, the above shown by the state of the art there is still a need to provide intravenous treatment devices for the treatment of varicose veins.

### BRIEF DESCRIPTION OF THE FIGURES

Figure 1 presents a view of the varicose vein treatment catheter device of the present invention.
Figure 2 presents a detail of one of the working ends of the catheter device.
Figure 3 presents a detail of the passage of fiber optics in the device of the present invention.
Figure 4 shows a detail of the ports through which the inflatable balloon device and the sclerosing agent are introduced.
Figures 5 and 8 present a detail of the working direction of the device on the patient's body.
Figures 6 and 7 present a detail of the windows or orifices and the agent coming out of them.
Figure 9 shows a detail of the incision in the patient's leg.

### DETAILED DESCRIPTION OF THE INVENTION

The device for the treatment of varicose veins of a patient of the present invention is composed of a central body with a plurality of windows or orifices, a working end towards the inner part of the patient's leg with an exit hole, a working end manipulated by medical personnel consisting of three entry routes to enter an inflatable balloon device, a fiber optic element and a sclerosing foaming agent.

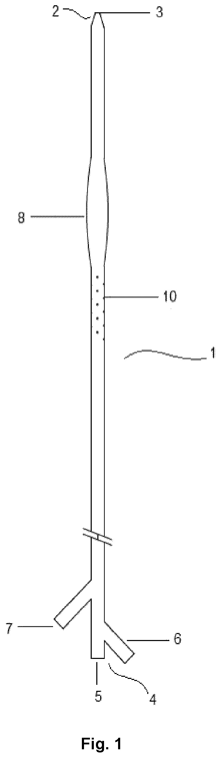

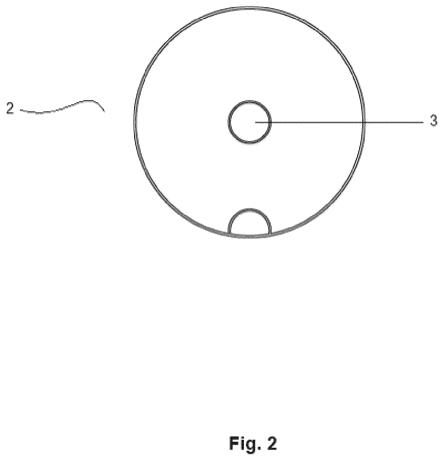

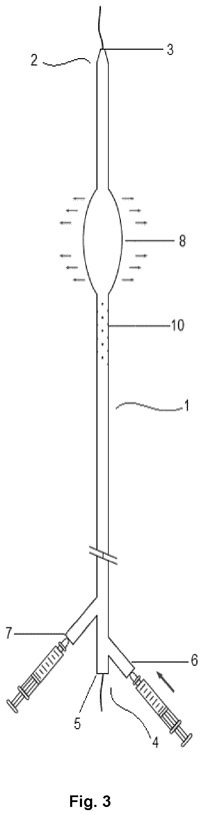

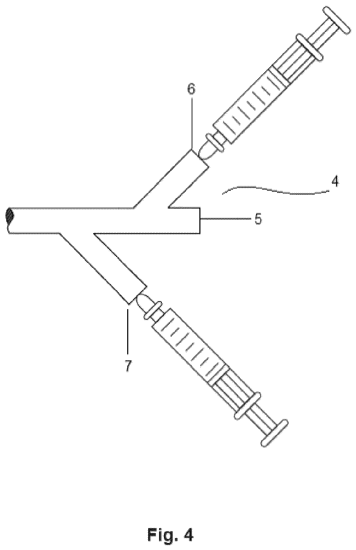

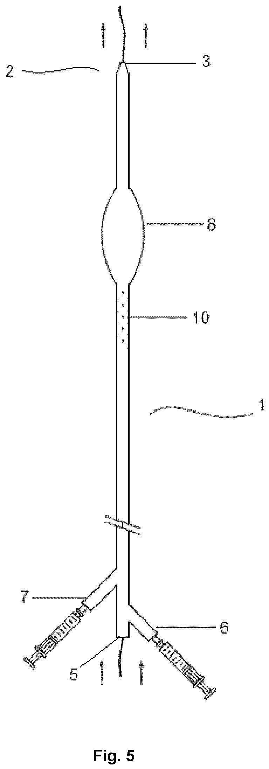

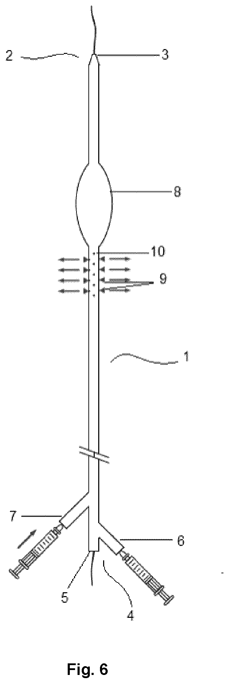

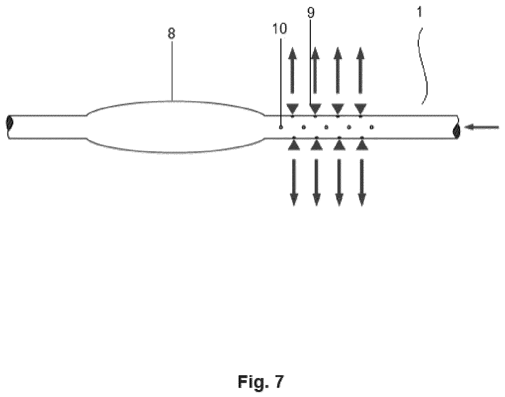

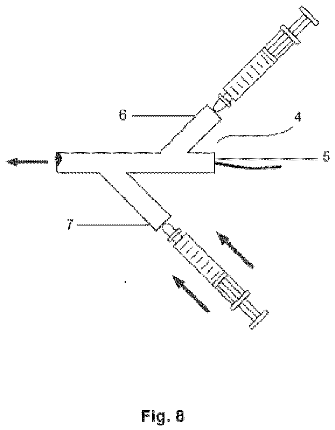

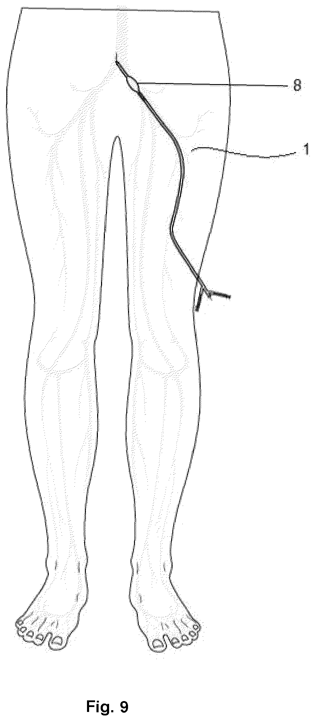

According to figures 1-9 presented, it is clear that the device of the present invention is of the intravenous type for the treatment of varicose veins which consists of a body (1), a working end (2) towards the inner part of the patient's leg with an exit hole (3), a working end (4) manipulated by medical personnel consisting of three routes of admission, the first of them (5) to enter a fiber optic element, two other ports (6, 7) that can enter an inflatable balloon device (8) and a sclerosing foaming agent such as polidocanol (9), the main body (1) also present a plurality of windows or orifices (10).

The window or orifice area (10) are less than 1 mm in diameter and are 50 mm apart from each other and are located behind the inflatable balloon device at a distance of 150 to 250 mm. The insufflation zone of the sclerosing foaming agent is located 50 to 100 mm in front of the inflatable balloon device area. This inflatable ball device has a diameter of between 20 and 40 mm.

Another object of the present invention is to provide a procedure to treat varicose veins in a patient using the device described above, this device is designed for those patients who suffer from a disease called chronic venous insufficiency or chronic venous disease; they are people who suffer from valve damage of the deep or superficial venous system; This device is designed to repair valve damage of the superficial venous system; In this system we refer primarily to two types of veins: the greater saphenous and the lesser saphenous, of these two, 95% of the time it is the greater saphenous that suffers damage, this device can also be used for the lesser saphenous, but preferentially it is focused on the greater saphenous. The damage is to the valves that are located inside the vein, usually at the junction between the superficial vein and the deep veins located in the groin of the patient's leg; this damage produces a chain reaction in which the following valves of the leg veins are affected because they cannot withstand the pressure and, when the person stands up, generates pressure over 120 mm of Hg; When the first valve is damaged, a pressure overload effect is generated on the other valves that are not designed to withstand this overload.

The increase in pressure generates symptoms such as tiredness and heaviness in the legs, cramps in the afternoon or night, in women there is generally discomfort during menstruation and it can be dramatically exacerbated during pregnancies due to the compression that the fetus generates on the veins of the legs. Over time, this evolution will produce edema and changes in color, because not only does water escape from the veins but also red blood cells that die in the tissue, producing this pigmentation, toxicity to the skin and can produce what is known as varicose ulcer. The device of the present invention is designed to prevent this situation by removing the diseased vein, preferably the greater saphenous vein. When this type of vein is damaged, other associated conditions are generated that are known as varicose veins.

What usually happens is that first the damaged saphenous vein is removed and then the affected varicose veins are removed; the differential that arises in relation to the state of the art is that, in a single use of the device, the occlusion of the saphenous trunk and, simultaneously, the obliteration of the varicose veins is performed so that with a single device, with a single puncture, two activities are carried out; the first is that under ultrasound visualization the saphenous occlusion is performed, where first the saphenous puncture is performed and a guidewire is passed, the needle is removed leaving the guidewire, an introducer is inserted or the catheter device (1) of the present invention is inserted directly, depending on the diameter obtained, which ideally is at least seven French (F) or 2.3 mm; the catheter device navigates over the said guidewire in such a way that the catheter (1) goes to the blood vessel, once there is blood inside the catheter, the guidewire is removed and, with the catheter (1) in position, the femoral saphenous junction is reached; at the working end of the catheter (4), an optical fiber is passed through port (5), this fiber element can be between 200 and 400 nm in diameter, and, under ultrasound, the progress to reach the femoral saphenous junction, that is, the groin, is located one or two cm behind the junction inside the superficial vein is supervised.

Tumescent anesthesia is then applied to perform a painless burn and for the vein to bind on the optical fiber, then the latter is removed.

Having eliminated the proximal segment of the saphenous membrane, the inflatable balloon element is inflated (8) behind this element are the windows (10) of the catheter through which the sclerosing foam is going to come out (9), this sclerosing agent is a surfactant that breaks the lipid walls of the endothelial cells of the inner lining of the vein. The insufflation of the balloon (8) is to prevent the sclerosing agent (9) from migrating to the proximal area, that is, up the patient's leg, the agent must migrate in the direction of the patient's feet; Subsequently, the sclerosing agent (9) is injected as a foam, after waiting between 2 and 4 minutes the balloon is deflated (8), the catheter device is removed (1) and the procedure is completed in the patient. At the same time and simultaneously, the patient's varicose veins are obliterated.

Although the present invention has been described with the preferential embodiments shown, it is understood that the modifications and variations which retain the spirit and scope of this invention are within the scope of the accompanying claims.

## Claims

1. An intravenous device for the treatment of saphenous veins and varicose veins **characterized by** comprising a body (1), a working end (2) towards the inner part of the patient's leg with an exit hole (3), a working end (4) manipulated by medical personnel consisting of three admission routes, the first of them (5) to enter a fiber optic element, two other ports (6, 7) to insert an inflatable balloon device (8) and a sclerosing foaming agent such as polidocanol (9), said main body (1) also have a plurality of windows or orifices (10).

2. **The** intravenous device for the treatment of varicose veins of claim 1 is **characterized in that** the windows or orifices are less than 1 mm in diameter, are 50 mm apart from each other and are located behind said inflatable balloon device at a distance of 150 to 250 mm.

3. **The** intravenous device for the treatment of varicose veins in claim 1 is **characterized by** the fact that the area of insufflation of the sclerosing foaming agent is 50 to 100 mm in front of the area of said inflatable balloon device.

4. **The** intravenous device for the treatment of varicose veins of claim 1 wherein the inflatable balloon device has a diameter of between 20 and 40 mm.

5. A process for treating saphenous vein and associated varicose veins in a patient using the device in claim 1 consisting of the following steps:
a) perform the puncture of the saphenous vein with a needle element and pass a guidewire, the needle is removed, leaving said guidewire, the catheter device (1) is inserted directly in a diameter or hole in the patient's leg of at least seven french (F) or 2.3 mm; the catheter device navigates over said guidewire in such a way that the catheter (1) goes to the blood vessel, once there is blood inside the catheter the guidewire is removed and, with the catheter (1) in position, the femoral saphenous junction is reached;
b) pass an optical fiber through port (5) at the working end of the catheter (4), this optical fiber element can be between 200 and 400 nm in diameter and, under ultrasound, the progress to reach the femoral saphenous junction is monitored, locating said fiber one or two cm behind the junction inside the superficial vein;
c) apply tumescent anesthesia to perform a painless burn and so that the vain is attached to the optical fiber, then the latter is removed;
d) having eliminated the proximal segment of the saphenous membrane, the inflatable balloon element is inflated (8), behind this element are the windows (10) of the catheter through which the sclerosing agent comes out (9);
e) inject the sclerosing agent (9) in foam form and wait for 2 to 4 minutes;
f) the balloon is deflated (8), the catheter device is removed (1) and the procedure is completed in the patient; and
g) at the same time and simultaneously, the patient's varicose veins are obliterated.

6. The process for treating varicose veins of claim 5 is **characterized by** the optical fiber transmitting a laser to cauterize the saphenous vein.
